# EUROPÄISCHE PATENTSCHRIFT

(11) **EP 2 736 570 B1**
(45) Veröffentlichungstag und Bekanntmachung des Hinweises auf die Patenterteilung: **01.08.2018**
(21) Anmeldenummer: 12727868.7
(22) Anmeldetag: 18.06.2012
(51) Int. Cl.: A61M 15/00, H02K 7/116, H02K 7/18, H02K 35/02, F16H 21/44, B05B 12/00, B05B 11/00

(54) **AUSTRAGVORRICHTUNG FÜR MEDIEN**
DISCHARGE DEVICE FOR MEDIA
DISPOSITIF D'EXTRACTION POUR MILIEUX

(30) Priorität: 27.07.2011 DE 102011079950
(43) Veröffentlichungstag der Anmeldung: 04.06.2014
(73) Patentinhaber: Aptar Radolfzell GmbH, 78315 Radolfzell (DE)
(72) Erfinder: HEISEL, Thomas, 78224 Singen (DE); KOHNLE, Jörg, 78056 Villingen-Schwenningen (DE)
(74) Vertreter: Patentanwaltskanzlei Cartagena
(86) Internationale Anmeldenummer: PCT/EP2012/061572
(87) Internationale Veröffentlichungsnummer: WO 2013/013892

(56) Entgegenhaltungen:
- EP-B1- 1 559 083
- WO-A1-2007/137991
- US-A- 2 239 717
- US-A1- 2007 017 506
- US-A1- 2007 135 756

## Beschreibung

Die Erfindung betrifft eine Austragvorrichtung für flüssige, pastöse oder pulverförmige Medien mit einem Gehäuse, mit einer Austragöffnung zum Austrag des Mediums, mit einem Reservoir zur Speicherung des Mediums vor dessen Austrag und mit einer gegenüber dem Gehäuse linearbeweglichen Handhabe, welche durch eine manuell bewirkte Hubbewegung die Förderung von Medium vom Reservoir zur Austragöffnung bewirkt. Dabei weist die Austragvorrichtung einen elektrischen Verbraucher auf. Weiterhin weist die Austragvorrichtung eine Umwandlungseinrichtung auf, die zur zumindest teilweisen Umwandlung der an der Handhabe eingebrachten mechanischen Energie in elektrische Energie zur Versorgung des elektrischen Verbrauchers ausgebildet ist.

Gattungsgemäße Austragvorrichtungen sind als Spender für kosmetische und pharmazeutische Medien bekannt. Es handelt sich üblicherweise um tragbare Spender, die insbesondere als Spender mit einem pharmazeutischen Medium befüllt sind und einem Patienten die Verabreichung dieses Mediums gestatten.

Bei Spendern wird es zunehmend üblich, dass diese einen elektrischen Verbraucher aufweisen, wobei es sich beispielsweise um einen elektronischen Zähler in Form eines Mikroprozessors und/oder elektronischer Anzeigemittel wie Displays oder LEDs handeln kann. Auch andere elektronische Komponenten wie beispielsweise Funk-Sendereinrichtungen oder Sperrmechanismen, die eine erneute Verabreichung eines Medikaments für einen bestimmten Zeitraum unterbinden, sind aus dem Stand der Technik bekannt. Zur Versorgung des elektrischen Verbrauchers ist die im Stand der Technik meist gewählte Lösung die eines bei Auslieferung geladenen Energiespeichers in Form eines Akkumulators oder einer Batterie.

Aus dem Stand der Technik sind jedoch ebenfalls Gestaltungen bekannt geworden, bei denen statt eines solchen vorgeladenen Energiespeichers Mittel zur Erzeugung elektrischer Energie in Form der eingangs genannten Umwandlungseinrichtungen vorgesehen sind. Diese nutzen die mechanische Energie, die durch den Benutzer durch Bewegen der Handhabe eingebracht wird und wandeln diese zum Teil in elektrische Energie um. Zwar ist die Energieausbeute hierbei gering. Bei entsprechender Auslegung der elektrischen Komponenten kann diese Energie jedoch ausreichen, um beispielsweise in Reaktion auf einen Austragvorgang ein elektronisches Zählwerk weiterzusetzen und den sich hierbei einstellenden Wert auf einem LC-Display anzuzeigen.

Aus der EP 1 559 083 B1 ist ein Spender bekannt, bei dem im Zuge der Betätigung ein Schlagbolzen in Betätigungsrichtung auf ein piezoelektrisches Element schlägt, um hierdurch elektrische Energie zu erzeugen. Die Verlagerung des Schlagbolzens speist sich dabei aus mechanischer Energie, die über die Betätigung des Spenders in diesen eingebracht wurde. Die Bewegung des Schlagbolzens ist hier eine Linearbewegung.

Aus der WO 2007/137991 A1 ist es bekannt, eine rotativ arbeitende Umwandlungseinrichtung zu verwenden. Die WO 2007/137991 A1 schlägt hierfür einen Generator vor, der über ein Ritzel verfügt, welches mittels einer linearbeweglichen Zahnstange angetrieben wird.

Diese bekannten Umwandlungseinrichtungen zur Erzeugung elektrischer Energie und deren Koppelung mit der jeweiligen Betätigungshandhabe werden nicht als ideal angesehen, da sie mit der Art der Bewegungsrichtung eine geringe Flexibilität bei der Auswahl der Umwandlungseinrichtung zur Folge haben. Auch ist die Ausbeute elektrischer Energie je nach Verbrauch der elektrischen Komponenten zu gering.

### Aufgabe und Lösung

Aufgabe der Erfindung ist es, eine gattungsgemäße Austragvorrichtung dahingehend weiterzubilden, dass diese eine verbesserte Möglichkeit zur Erzeugung elektrischer Energie gestattet.

Gemäß dem Hauptaspekt der vorliegenden Erfindung wird dies dadurch erreicht, dass zur Zuführung der an der Handhabe eingebrachten mechanischen Energie zur Umwandlungseinrichtung ein Getriebe vorgesehen ist, mittels dessen die lineare Bewegung der Betätigungshandhabe oder eines Spanngliedes eines mittels der Handhabe spannbaren Federenergiespeichers in eine nicht rotative oszillierende Bewegung eines Pendelgliedes umgewandelt wird, wobei die Umwandlungseinrichtung zur mittelbaren oder unmittelbaren Umwandlung der mechanischen Energie des oszillierenden Pendelgliedes in elektrische Energie ausgebildet ist.

Eine erfindungsgemäße Austragvorrichtung weist somit entsprechend einer gattungsgemäßen Austragvorrichtung die für den unmittelbaren Austrag des Mediums erforderlichen Komponenten in Form des Reservoirs und einer Austragöffnung auf, wobei das Medium durch die linearbewegliche Handhabe, vorzugsweise mittels einer mit der Handhabe gekoppelten Pumpe, vom Reservoir zur Austragöffnung gefördert werden kann. Neben der Pumpeinrichtung sind jedoch auch andere Gestaltungen denkbar, beispielsweise eine solche bei der das Medium vor dem Austrag bereits unter Druck steht und bei der ein Auslassventil im Verbindungskanal zwischen Reservoir und Austragöffnung bei Betätigung der Handhabe geöffnet wird. Zusätzlich zu diesen für Austragvorrichtungen allgemein bekannten Merkmalen ist der genannte mindestens ein elektrischer Verbraucher vorgesehen, der beispielsweise als Display, als LED oder als Mikroprozessor ausgebildet sein kann. Vorzugsweise sind mehrere elektrische Verbraucher vorgesehen, die verschiedene Funktionen im Zusammenspiel erfüllen. Die in allgemeiner Form bereits von gattungsgemäßen Spendern bekannte Umwandlungseinrichtung versorgt den mindestens einen elektrischen Verbraucher mit elektrischer Energie, wobei diese Versorgung vorzugsweise nicht unmittelbar erfolgt. Stattdessen können Komponenten zur Anpassung der Spannung vorgesehen sein. Auch eine Speichereinrichtung wie beispielsweise ein Kondensator zur kurzzeitigen Zwischenspeicherung der elektrischen Energie kann vorgesehen sein. Die erfindungsgemäße Austragvorrichtung kann weiterhin auch über einen Akkumulator zur längerfristigen Speicherung elektrischer Energie verfügen. Dieser kann bei einer solchen Gestaltung unmittelbar zur Versorgung des mindestens einen elektrischen Verbrauchers genutzt werden und seinerseits durch die Umwandlungseinrichtung aufgeladen werden.

Die Umwandlungseinrichtung ist gemäß dem ersten Aspekt der Erfindung zur Umwandlung der mechanischen Energie eines oszillierenden Pendelgliedes in elektrische Energie ausgebildet. Dieses Pendelglied ist mittels eines Getriebes derart mit der Handhabe gekoppelt, dass es während einer Hubbewegung der Handhabe mehrfach zwischen zwei Endlagen hin- und herbewegt wird. Eine solche Pendelbewegung gestattet die Verwendung von Umwandlungseinrichtungen, welche bei unmittelbarer nicht oszillierender Ankoppelung an die Betätigungshandhabe keine ausreichende Menge Energie liefern würden. Insbesondere sind Umwandlungseinrichtungen bei einer erfindungsgemäßen Gestaltung verwendbar, die eine oszillierende Linearbewegung in elektrische Energie umwandeln.

Das Getriebe kann derart an das Pendelglied angekoppelt sein, dass es in Reaktion auf eine Hubbewegung der Handhabe sowohl die Bewegung des Pendelgliedes in Richtung einer ersten Endlage als auch die anschließende Bewegung des Pendelgliedes in die gegenüberliegende zweite Endlage erzwingt. Es kann jedoch auch zweckmäßig sein, das Pendelglied durch das Getriebe lediglich in eine Richtung auszulenken und die nachfolgende entgegengesetzte Bewegung des Pendelgliedes mittels einer dem Pendelglied zugeordneten Federeinrichtung zu bewirken. Bei einem Pendelglied, welches für eine oszillierende Biegeauslenkung in Reaktion auf eine Betätigung der Handhabe ausgebildet ist, kann diese Federeinrichtung auch durch die Elastizität des Pendelgliedes selbst zur Verfügung gestellt werden.

Das Getriebe kann unmittelbar die Bewegung der Betätigungshandhabe in die Bewegung des Pendelgliedes überführen, so dass eine Verlangsamung der Betätigung der Handhabe auch eine Verlangsamung der Bewegung des Pendelgliedes bewirkt. Da je nach Umwandlungseinrichtung hohe Bewegungsgeschwindigkeiten des Pendelgliedes und/oder eine bestimmte Geschwindigkeit des Pendelgliedes gewünscht sein kann, ist es bei einer Weiterbildung jedoch auch möglich, dass der Federenergiespeicher mit dem gegenüber dem Gehäuse beweglichen und oben bereits genannten Spannglied und einer zwischen dem Gehäuse und dem Spannglied wirkenden Feder ausgebildet ist. Dabei ist vorgesehen, dass das Spannglied während der Hubbewegung mit der Handhabe wirkgekoppelt ist, so dass ein Spannen der Feder bewirkt wird, dass weiterhin das Spannglied im gespannten Zustand der Feder von der Handhabe automatisch entkoppelt wird, so dass ein Entspannen der Feder bewirkt wird, und dass das Spannglied während des Entspannens der Feder über das Getriebe mit dem Pendelglied zumindest phasenweise wirkgekoppelt ist, so dass die Bewegung des Spanngliedes die oszillierende Bewegung des Pendelgliedes bewirkt. Durch diese Gestaltung wird erreicht, dass die oszillierende Bewegung des Pendelgliedes zumindest phasenweise vollständig von der konkreten Art der Betätigung durch den Benutzer unabhängig ist. Der Benutzer spannt während der Hubbewegung der Handhabe eine Feder. Sobald diese gespannt ist, kommt es, vorzugsweise bei einer definierten Relativlage der Handhabe zum Gehäuse, zu einer Entkoppelung des Spanngliedes von der Handhabe, so dass das Spannglied in reproduzierbarer Weise durch die Federenergie der Feder angetrieben zurückbewegt wird und dabei über das Getriebe auf das oszillierende Pendelglied wirkt. Da die Bewegung des Spanngliedes von der benutzerabhängigen Betätigungsbewegung unabhängig ist, kann somit eine oszillierende Bewegung in für die Umwandlungseinrichtung idealer Geschwindigkeit bewirkt werden. Alternativ zu dieser Gestaltung, bei der das Spannglied während der Hubbewegung mit der Handhabe gekoppelt ist, ist es auch möglich, das Spannglied während der Hubbewegung ortsfest zum Gehäuse zu fixieren und die Feder zwischen dem Spannglied und der Handhabe vorzusehen, so dass es ebenfalls zu einem Spannen der Feder während der Hubbewegung der Handhabe kommt. In einem solchen Falle wird bei gedrückter Handhabe eine Entkoppelung des Spanngliedes vom Gehäuse und damit die gewünschte benutzerunabhängige Bewegung des Spanngliedes verursacht. Da das Gehäuse und die Handhabe lediglich dadurch definiert sind, dass ihre Relativbewegung den Austragvorgang bewirkt, können die Begriffe frei gegeneinander ausgetauscht werden.

Für die Ausgestaltung des Getriebes sind ist eine Reihe von Gestaltungen denkbar. Eine besonders einfache mögliche Gestaltung sieht vor, dass die Linearbewegung der Betätigungshandhabe über eine Zahnstange in eine Drehbewegung eines Zahnrades überführt wird. Dessen Drehbewegung kann dann über eine Kolbenstange auf ein linearbewegliches Pendelglied weitergeleitet werden, welches mit der Umwandlungseinrichtung gekoppelt ist.

Eine andere Gestaltung sieht vor, dass das Pendelglied drehbar oder im Sinne einer Biegebewegung auslenkbar gelagert ist und mit einer Verzahnung oder Profilierung an der Handhabe, am Spannglied oder an einem mit der Handhabe oder mit dem Spannglied in Wirkverbindung stehenden Zwischenglied derart zusammenwirkt, dass die Bewegung der Verzahnung oder Profilierung je Zahn der Verzahnung oder je Profilabschnitt der Profilierung dazu führt, dass das Pendelglied in Eingriff mit der Verzahnung oder Profilierung gelangt, durch diese vorzugsweise elastisch ausgelenkt oder gegen die Kraft einer Feder gedreht wird und anschließend wieder außer Eingriff mit der Verzahnung oder Profilierung kommt, so dass es in seine Ausgangslage zurückspringt, um vom nächsten Zahn oder der nächsten Profilierung in gleicher Weise erneut bewegt zu werden.

Bei einer solchen Gestaltung wird somit das Pendelglied aus einer Ruhelage wiederholt von der Profilierung oder Verzahnung ausgelenkt, bis diese den Kontakt zum Pendelglied verliert und dieses infolgedessen durch eine externe Federkraft oder seine Eigenelastizität in die Ruhelage zurückspringt. Dort wird es anschließend von der nächsten Verzahnung oder Profilierung ergriffen. Eine solche Ausgestaltung ist baulich recht einfach und gestattet bei der Verwendung eines Piezo-Biegebalkens als Umwandlungseinrichtung eine sehr unmittelbare Verwendung der oszillierenden Bewegung des Pendelgliedes zur Erzeugung elektrischer Energie.

Eine weitere Gestaltung eines erfindungsgemäßen Getriebes sieht vor, dass an der Handhabe, am Spannglied oder an einem mit der Handhabe oder mit dem Spannglied in Wirkkopplung stehenden Zwischenglied eine Kurvenbahn vorgesehen ist, an der ein am Pendelglied vorgesehener Fortsatz anliegt, so dass durch eine Bewegung der Handhabe oder des Spanngliedes die Kurvenbahn bewegt wird und der Fortsatz entlang der Kurvenbahn verlagert wird und dadurch die Pendelbewegung des Pendelgliedes bewirkt.

Bei einer solchen Gestaltung folgt ein an der Kurvenbahn anliegender oder mit einer als Kulissenbahn ausgebildet Kurvenbahn in Eingriff befindlicher Teil des Pendelgliedes aufgrund einer vorgesehenen begrenzten Beweglichkeit des Pendelgliedes, vorzugsweise einer Linearbeweglichkeit des Pendelgliedes, in einer von der Bewegungsrichtung der Handhabe, des Spanngliedes oder des Zwischengliedes abweichenden Richtung somit der Kurvenbahn. Diese kann beispielsweise die Form einer Sinuskurve aufweisen und dadurch das Pendelglied wiederholt hin- und herbewegen. Die Kurvenbahn kann als gewellte Kante eines Kurvenbahnbauteils vorgesehen sein, gegen die der Fortsatz des Pendelgliedes mittels einer dem Pendelglied zugeordneten Feder gedrückt wird. Diese Variante ermöglicht eine besonders einfache Montage. Die Kurvenbahn kann jedoch auch als nutartige Kulissenbahn ausgebildet sein, in die ein Fortsatz des Pendelgliedes eingreift und die somit ohne eine zusätzliche Feder am Pendelglied dieses hin- und herbewegt.

Was die Gestaltung der Umwandlungseinrichtung zur Erzeugung elektrischer Energie angeht, so sind hier viele Alternativen anwendungsfallspezifisch zweckmäßig.

Eine bevorzugte Ausgestaltung sieht vor, dass die Umwandlungseinrichtung als elektromagnetischer Generator ausgebildet ist, der eine Komponente mit einem Magneten und eine Komponente mit einem mit dem elektrischen Verbraucher verbundenen Leiter, vorzugsweise in Form einer Spule, aufweist, wobei eine der Komponenten gehäusefest ist und wobei die andere Komponente am Pendelglied vorgesehen ist. Ein solcher elektromagnetischer Generator kann durch das Pendelglied und dessen oszillierende Bewegung elektrische Energie für den Verbraucher zur Verfügung stellen.

Eine alternative Gestaltung einer Umwandlungseinrichtung, die auch im Zusammenhang mit einer gattungsgemäßen Austragvorrichtung und somit ohne oszillierendes Pendelglied offenbart ist, weist die folgenden Komponenten auf. Sie verfügt über ein Induktionsorgan, welches länglich ausgebildet ist und zwischen einem ersten Endbereich und einem zweiten Endbereich von einer Spule umwickelt ist. Weiterhin verfügt diese Umwandlungseinrichtung über eine Magneteinheit mit einem Magneten und einer ersten und einer zweiten auf Seiten des Magnet-Nordpols vorgesehenen Nordpolfläche und einer ersten und einer zweiten auf Seiten des Magnet-Südpols vorgesehenen Südpolfläche. Dabei sind das Induktionsorgan und die Magneteinheit gegeneinander zwischen einer ersten Relativposition und einer zweiten Relativposition beweglich, wobei in der ersten Relativposition der erste Endbereich des Induktionsorgans an der ersten Nordpolfläche und der zweite Endbereich des Induktionsorgans an der zweiten Südpolfläche anliegt und in der zweiten Relativposition der erste Endbereich des Induktionsorgans an der ersten Südpolfläche und der zweite Endbereich des Induktionsorgans an der zweiten Nordpolfläche anliegt. Dabei werden durch Betätigen der Handhabe das Induktionsorgan und die Magneteinheit während eines Betätigungshubes einfach oder mehrfach zwischen den Relativpositionen verlagert.

Bei einer Ausgestaltung mit einem oszillierenden Pendelglied ist dieses mit der Magneteinheit oder dem Induktionsorgan derart gekoppelt, dass die Magneteinheit und das Induktionsorgan in Reaktion auf eine Hubbewegung der Handhabe mehrfach hin- und her verlagert werden. Eine beschriebene Umwandlungseinrichtung eignet sich jedoch auch zu Austragvorrichtungen, bei denen in Reaktion auf eine Bewegung der Handhabe lediglich eine einfache Relativverlagerung von Induktionsorgan und Magneteinheit stattfindet, gegebenenfalls mit einer einfachen Rückbewegung, die durch eine hierfür vorgesehene Feder bewirkt werden kann. Das Wirkprinzip der genannten Umwandlungseinrichtung mit Induktionsorgan und Magneteinheit basiert darauf, dass das magnetische Induktionsorgan mit seinen beiden Endbereichen im Wechsel magnetisch umgepolt wird. In der ersten Relativposition ist der erste Endbereich in Kontakt mit dem Nordpol, während der zweite Endbereich in Kontakt mit dem Südpol der Magneteinheit ist. In der zweiten Relativposition ist der erste Endbereich in Kontakt mit dem Südpol, während der zweite Endbereich in Kontakt mit dem Nordpol ist. Diese gegebenenfalls während einer Betätigung der Handhabe mehrfach stattfindende Umpolung des Induktionsorgans induziert eine Spannung in die Spule, welche unmittelbar oder mittelbar zur Bestromung des elektrischen Verbrauchers verwendet werden kann. Dabei ist nicht zwingend ein vollständiges Anliegen des Induktionsorgans an den jeweiligen Flächen erforderlich. Auch durch eine wechselnde Annäherung an die jeweiligen Flächen lässt sich eine vergleichbare Wirkung erzielen.

Bei einer weiteren Variante einer Umwandlungseinrichtung, welche ebenfalls im Zusammenhang mit dem oben genannten oszillierenden Pendelglied, jedoch auch mit einer gattungsgemäßen Austragvorrichtung, verwendet werden kann, handelt es sich um einen einseitig gehäusefest eingespannten Piezobiegewandler, dessen freies Ende durch Betätigung der Handhabe während eines Betätigungshubes einfach oder mehrfach ausgelenkt wird. Ein solcher Piezobiegewandler, der für die erfindungsgemäße Verwendung bipolar oder unipolar ausgebildet sein kann, hat eine längliche Formgebung und besteht aus Piezokristallen bzw. weist solche Piezokristalle auf, die in diesem Falle auf einem Träger des Biegewandlers aufgebracht sind. Diese Piezokristalle sind dabei so angeordnet, dass eine Verbiegung des einseitig eingespannten Piezobiegewandlers quer zu seiner Haupterstreckungsrichtung die Erzeugung einer elektrischen Spannung bewirkt. Ein solcher Piezobiegewandler kann durch das Getriebe in Reaktion auf einen Betätigungshub einfach oder mehrfach ausgelenkt und zurückgelenkt werden.

Besonders von Vorteil ist es, wenn die Eigenfrequenz des Piezobiegewandlers hierbei Berücksichtigung findet. So kann der Piezobiegewandler insbesondere als in Reaktion auf eine manuelle Betätigung der Handhabe oszillierend bewegtes Pendelglied ausgebildet sein, wobei die oszillierende Bewegung durch den Federenergiespeicher mit mechanischer Energie versorgt wird und wobei die Feder des Federenergiespeichers derart an den Piezobiegewandler angepasst ist, dass dieser zumindest phasenweise mit seiner Resonanzfrequenz (± 20%) oszilliert. Bei einer solchen Gestaltung ist somit die oben beschriebene Entkoppelung der Bewegung des Pendelgliedes von der Bewegung der Betätigungshandhabe vorgesehen, so dass das Pendelglied unabhängig von einer schnellen oder langsamen Bewegung der Betätigungshandhabe mit einem immer gleichen Geschwindigkeitsprofil angeregt wird. Dieses kann auf die genannte Resonanzfrequenz hin eingestellt sein, so dass eine besonders große Energieausbeute erzielt wird.

Wie eingangs bereits erwähnt wurde, handelt es sich bei der Bewegung des Pendelgliedes vorzugsweise um eine oszillierende Linearbewegung. Diese oszillierende Linearbewegung erfolgt vorzugsweise in einem rechten Winkel zur Betätigungsrichtung der Handhabe. Insbesondere in einem solchen Falle, jedoch auch bei anderweitigen oszillierenden Bewegungen des Pendelgliedes, wird es als vorteilhaft angesehen, wenn die Handhabe entlang einer Betätigungsrichtung gegenüber dem Gehäuse beweglich ist, die mit einer durch eine Austragrichtung des Mediums definierten Haupterstreckungsrichtung der Austragvorrichtung einen Winkel zwischen 70° und 110° einschließt.

### Kurzbeschreibung der Zeichnungen

Weitere Aspekte und Vorteile der Erfindung ergeben sich außer aus den Ansprüchen auch aus der nachfolgenden Beschreibung von bevorzugten Ausführungsbeispielen der Erfindung, die nachfolgend anhand der Figuren erläutert werden. Dabei zeigen:
- Fig. 1a und 1b: eine erfindungsgemäße Austragvorrichtung im unbetätigten und im betätigten Zustand mit einer ersten Variante eines Getriebes und einer ersten Variante einer Umwandlungseinrichtung zur Erzeugen elektrischer Energie,
- Fig. 2 bis 5: verschiedene Varianten eines Getriebes für die Austragvorrichtung der Fig. 1a und 1b und
- Fig. 6 bis 8: verschiedene Varianten einer Umwandlungseinrichtung für die Austragvorrichtung der Fig. 1a und 1b.

### Detaillierte Beschreibung der Ausführungsbeispiele

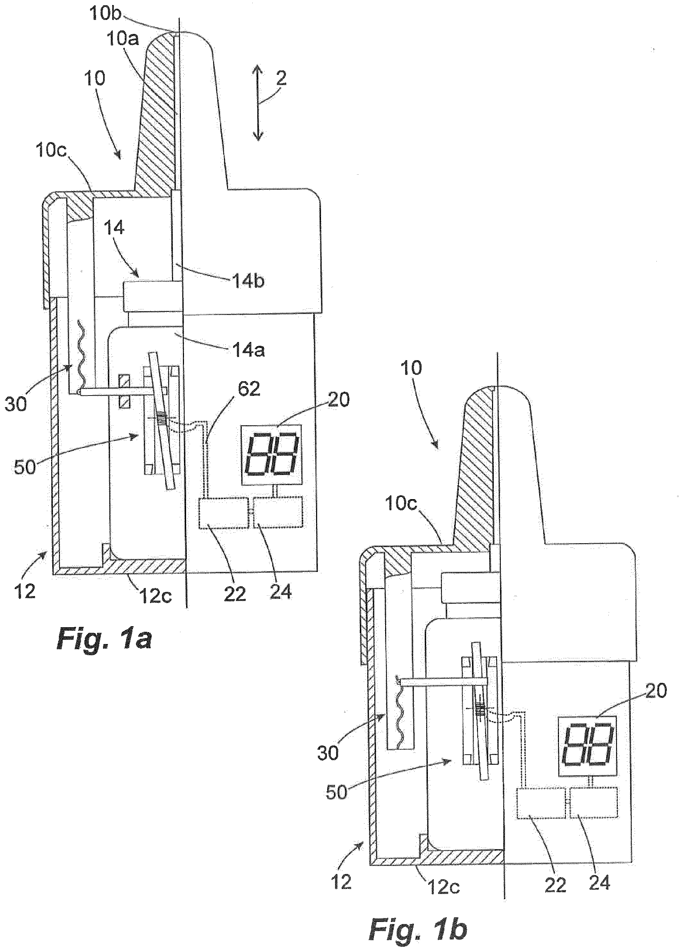

Die Fig. 1a und 1b zeigen in schematisch vereinfachter Form eine erfindungsgemäße Austragvorrichtung.

Diese umfasst zwei gegeneinander in Richtung des Pfeils 2 linear beweglich verlagerbare Gehäuseteile 10, 12, wobei der obere Gehäuseteil 10 ein Austragkanal 10a sowie eine Austragöffnung 10b aufweist. In einem von den Gehäuseteilen 10, 12 umschlossenen Gehäuseinnenraum ist ein Pumpspender 14 mit einem Mediumspeicher 14a sowie einem Auslassstutzen 14b angeordnet. Der Auslassstutzen 14b ist dabei so positioniert, dass er in den Auslasskanal 10a mündet. Ein Austragvorgang ist mit der dargestellten Austragvorrichtung erzielbar, indem die beiden Gehäuseteile 10, 12 in der in Fig. 1b skizzierten Weise durch Druckbeaufschlagung an Betätigungsflächen 10c, 12c gegeneinander kraftbeaufschlagt und so ineinandergedrückt werden. Dies verursacht, dass der Auslassstutzen 14b in Richtung des Reservoirs 14a des Pumpspenders 14 eingedrückt wird, wodurch ein nicht dargestelltes Auslassventil des Pumpspenders 14 geöffnet und das unter Druck stehende Medium aus dem Speicher 14a durch den Auslassstutzen 14b hindurch zur Austragöffnung 10b gefördert wird. Bei der vorliegenden Ausgestaltung ist somit vorgesehen, dass das Medium bereits in druckbeaufschlagter Form vorliegt und dass die Betätigung eine Austragung des Mediums durch Öffnen des Auslassventils verursacht. Dieses Beispiel ist jedoch rein exemplarisch. Gleichermaßen könnte auch eine Ausgestaltung vorgesehen sein, bei der die Verlagerung der Gehäuseteile 10, 12 zu einer Betätigung einer Pumpvorrichtung führt, die drucklos gespeichertes Medium zur Austragöffnung 10b fördert.

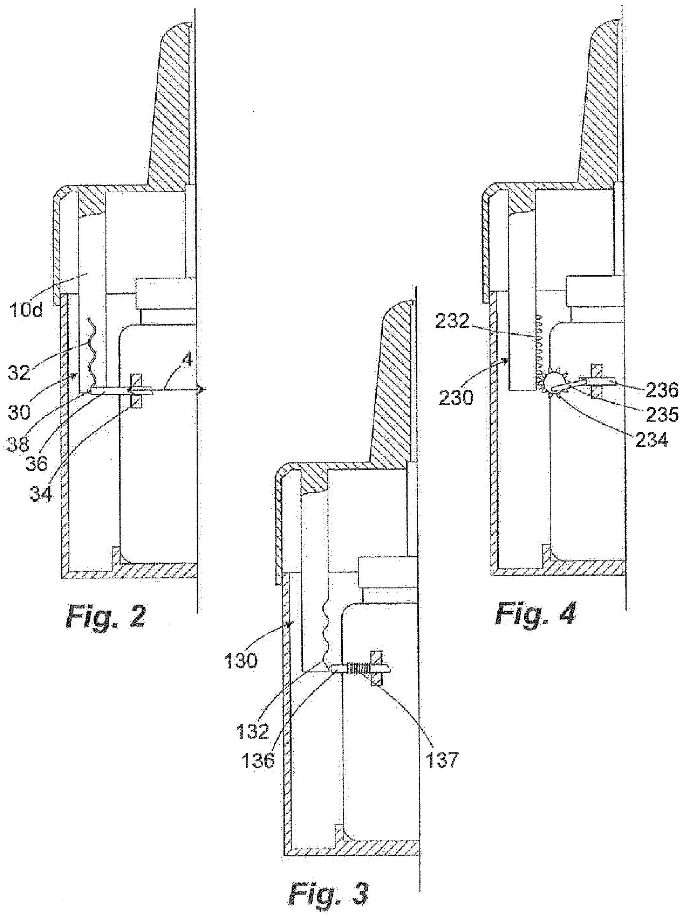

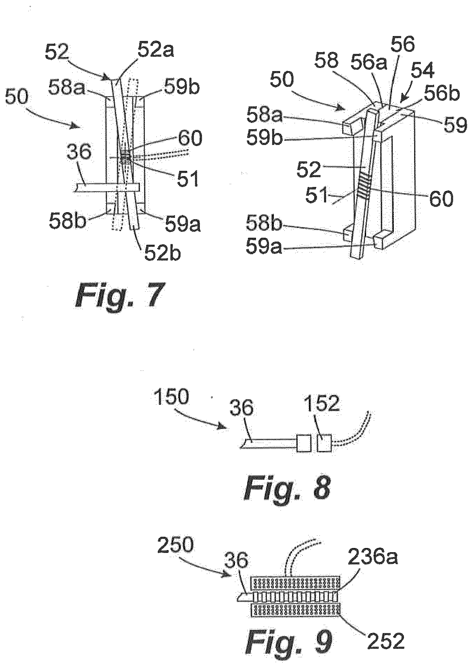

Die dargestellte Austragvorrichtung ist mit einem Zählwerk ausgestattet. Sie verfügt hierzu über ein LC-Display 24, welches mit einem lediglich angedeuteten Mikroprozessor 20 verbunden ist. Der Mikroprozessor 20 und das Display 24 werden von einem als Kondensator ausgebildeten Pufferspeicher 22 mit elektrischer Energie versorgt. Eine Batterie zur Versorgung der elektronischen Komponenten 20, 24 ist nicht vorgesehen. Statt dessen ist der dargestellte Spender dafür ausgebildet, die zum zumindest kurzzeitigen Betrieb dieser elektronischen Komponenten erforderliche elektrische Energie aus jener mechanischen Energie zu gewinnen, die der Benutzer bei der Betätigung der Austragvorrichtung in das System einbringt. Zu diesem Zweck verfügt die Austragvorrichtung über ein Getriebe 30, welche das obere Gehäuseteil 10 mit einer Umwandlungseinrichtung 50 zur Erzeugung elektrischer Energie koppelt. Das Getriebe 30 sowie hierzu alternative Getriebe 130, 230, 330, 430 sind in den Fig. 2 bis 6 isoliert dargestellt. Alle diese Getriebe können gleichermaßen bei der Austragvorrichtung der Fig. 1a und 1b Verwendung finden. Die Umwandlungseinrichtung 50 sowie hierzu alternative Umwandlungseinrichtungen 150 und 250 sind in den Fig. 8 bis 9 dargestellt. Alle diese Umwandlungseinrichtungen können gleichermaßen bei der Austragvorrichtung der Fig. 1a und 1b Verwendung finden. Auch beliebige Kombinationen der Umwandlungseinrichtungen und der Getriebe sind möglich und von der Erfindung umfasst.

Die beim Ausführungsbeispiel der Fig. 1a und 1b verwendete Gestaltung ist die Folgende: Das Getriebe 30, welches separat nochmals in Fig. 2 dargestellt ist, umfasst einen am oberen Gehäuseteil 10 vorgesehenen Fortsatz 10d, der sich in den Innenraum der Austragvorrichtung erstreckt. An diesem ist eine als Nut ausgebildete Kulissenbahn 32 vorgesehen, welche vorliegend die Form einer Sinuskurve beschreibt. Das Getriebe 30 umfasst weiterhin ein durch eine am unteren Gehäuseteil 12 befestigte Lagerführung 34 lediglich orthogonal zur Richtung 2 linearbewegliches Pendelglied 36. Am der Kulissenbahn 32 zugewandten Ende weist das Pendelglied 36 einen Eingriffsstift 38 auf, der in die Kulissenbahn 32 hineinragt.

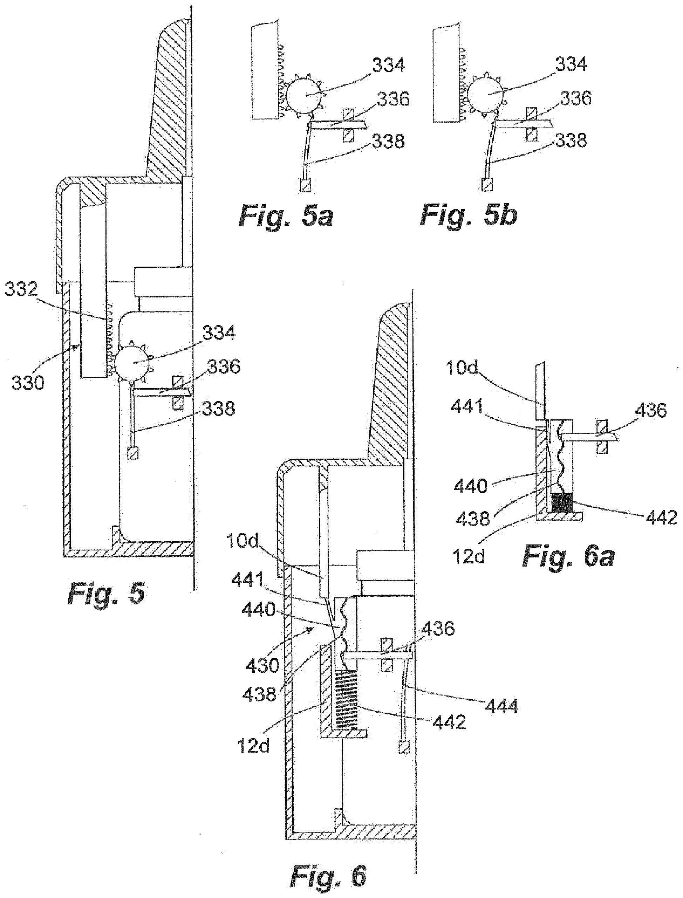

Hierdurch wird bewirkt, dass das Pendelglied beim Hinabdrücken des oberen Gehäuseteils 10 eine oszillierende Bewegung in Richtung des Pfeils 4 durchführt und so beim Ausführungsbeispiel insgesamt dreimal zwischen zwei Endlagen hin- und her oszilliert. Das Pendelglied 36 wiederum ist mit der Umwandlungseinrichtung 50 zur Erzeugung elektrischer Energie gekoppelt, welche in Fig. 6 genauer dargestellt ist.

Diese Umwandlungseinrichtung 50 verfügt über ein stabförmiges Induktionsorgan 52, welches um die Achse 51 schwenkbeweglich gelagert ist. Dieses Induktionsorgan ist benachbart zu einer Magneteinheit 54 angeordnet, welche über einen Permanentmagneten 56 mit einer Nordpolseite 56a und einer Südpolseite 56b verfügt. An diesen beiden Seiten 56a, 56b schließt sich jeweils ein plattenförmiger Körper 58, 59 aus magnetisierbarem Material an. Jede dieser Platten 58, 59 weist zwei Nordpol- bzw. Südpolfortsätze 58a, 58b, 59a, 59b auf. Diese limitieren die Bewegung des Induktionsorgans 52 zwischen zwei Endlagen.

In der in der linken Darstellung der Fig. 7 nicht gestrichelt dargestellten Endlage des Induktionsorgans 52 liegt dieses mit einem oberen Ende 52a am Nordpol-Fortsatz 58a und mit seinem unteren Ende 52b am Südpol-Fortsatz 59a an. Es wird daher entsprechend magnetisiert. Wenn eine Verlagerung in die entgegengesetzte gestrichelt dargestellte Endlage erfolgt, liegt in dieser das obere Ende 52a am Südpol-Fortsatz 59b an, während das untere Ende 52b am Nordpolfortsatz 58b anliegt. In dieser zweiten Endlage ist die Magnetisierung des Induktionsorgans 52 somit der Magnetisierung in der ersten Endlage entgegengesetzt. Dieser Wechsel der Magnetisierung des Induktionsorgans 52 wird mittels einer das Induktionsorgan 52 umgebenden Spule 60 genutzt, um in diese einen Strom zu induzieren. Dieser wird über Leitungen 62 zum Pufferspeicher 22 geleitet. Gegebenenfalls können hierzwischen noch ein Gleichrichter oder andere zur Umwandlung des Stroms geeignete Teileinrichtungen vorgesehen sein.

Die Kopplung zwischen dem Getriebe 30 und der Umwandlungseinrichtung 50 ist dergestalt ausgebildet, dass das Pendelglied 36 die genannte Bewegung des Induktionsorgans 52 zwischen seine beiden Endlagen bewirkt. Hierdurch erfolgt im Zuge eines Betätigungsvorgangs der Austragvorrichtung eine mehrfache Ummagnetisierung des Induktionsorgans 52 und dadurch eine Gewinnung elektrischer Energie, um hierdurch die genannten elektronischen Komponenten mit ausreichend Energie zu versorgen. Diese kann beispielsweise genutzt werden, um mittels des Mikroprozessors 20 ein Zählregister hochzusetzen und den entsprechenden Wert kurzzeitig oder im Falle eines bi-stabilen Displays dauerhaft bis zur nächsten Betätigung im LC-Display 24 anzuzeigen.

Die Fig. 3 bis 6 beschreiben alternative Gestaltungen des Getriebes, die mit der Umwandlungseinrichtung 50 oder den nachfolgend noch beschriebenen anderen Umwandlungseinrichtungen 150, 250 oder anderen Umwandlungseinrichtungen Verwendung finden können.

Die Ausgestaltung der Fig. 3 zeigt abweichend von der Ausgestaltung der Fig. 2 am Getriebe 130 eine einseitig offene Kurvenbahn 132. Um das Pendelglied 136 dennoch oszillierend zu bewegen, ist diesem eine Rückstellfeder 137 zugeordnet. Bezogen auf Fig. 3 wird somit die Bewegung des Pendelglieds nach rechts durch die Kurvenbahn 132 erzwungen, während die Bewegung nach links durch die Feder 137 bewirkt wird.

Bei der Ausgestaltung der Fig. 4 ist statt einer Kurvenbahn eine Zahnstange 232 am oberen Gehäuseteil 10 ortsfest vorgesehen, welche mit einem Zahnrad 234 kämmt. Dieses Zahnrad ist über eine exzentrisch angebrachte Kolbenstange 235 mit dem Pendelglied 236 verbunden. Über eine entsprechende Anbringung der Kolbenstange am Zahnrad 234 kann die Amplitude der Bewegung des Pendelgliedes 236 eingestellt werden. Die Frequenz kann über die Auswahl eines kleineren oder größeren Zahnrades 234 beeinflusst werden.

Beim Getriebe 330 der Fig. 5 ist wiederum eine mit dem Gehäuseteil 10 verbundene Zahnstange 332 vorgesehen, welche wiederum mit einem Zahnrad 334 kämmt. Die Ankopplung an das ebenfalls linearbewegliche Pendelglied 336 erfolgt bei dieser Ausgestaltung jedoch über einen mit seinem unteren Ende am unteren Gehäuseteil 12 eingespannten Biegebalken 338. Der in Fig. 5 dargestellten Position wird dieser Biegebalken 338 beim Hinabdrücken des oberen Gehäuseteils 10 von einem Zahn des Zahnrades 334 ergriffen und in der in Fig. 5a dargestellten Weise ausgelenkt, wobei es zu einer Biegeverformung des Biegebalkens 338 kommt. In dem in Fig. 5b dargestellten Zustand gelangt der Biegebalken 338 dann außer Eingriff mit dem genannten Zahn und kehrt aufgrund seiner Elastizität in die Lage der Fig. 5 zurück, wo er anschließend vom nächsten Zahn des Zahnrades 334 wieder erfasst wird. Das am Biegebalken 338 angebrachte Pendelglied 336 folgt dieser Bewegung. Durch Materialeigenschaften des Auslenkabschnitts 338 kann bei dieser Gestaltung beeinflusst werden, mit welcher Geschwindigkeit die rückschnellende Bewegung zwischen dem Zustand der Fig. 5b und dem Zustand der Fig. 5 abläuft. Durch ein hohes E-Modul kann eine sehr schnelle und schlagartige Rücküberführung erzielt werden, welche sich insbesondere für die Anbindung an eine piezoelektrische Umwandlungseinrichtung eignet.

Bei einer Variante der Ausführungsform der Fig. 5 kann der Biegebalken 338 selbst auch die Umwandlungseinrichtung darstellen, indem er als Piezo-Biegebalken ausgebildet ist. Dessen wiederholte Auslenkung führt zu einer piezoelektrischen Erzeugung elektrischer Energie. Bei einer solchen Gestaltung bildet der Biegebalken 338 selbst das Pendelglied. Das Pendelglied 336 entfällt.

Die Ausgestaltung der Fig. 6 ähnelt der Ausgestaltung der Fig. 1 und 2. Auch hier ist eine Kurvenbahn 438 vorgesehen, mit der in gleicher Art und Weise wie zu Fig. 2 beschrieben ein Pendelglied 436 zusammenwirkt. Die Besonderheit bei dieser Ausgestaltung liegt darin, dass die Kulissenbahn 438 nicht fest mit dem oberen Gehäuseteil 10 verbunden ist. Während des Niederdrückens des Gehäuseteils 10 wirkt der Steg 10d zwar aufgrund eines Kopplungsarms 441 an einem vertikal beweglichen Spannglied 440 mit der Kulissenbahn 438 zusammen und führt diese mit. Gleichzeitig wird aber beim Hinabdrücken eine dem Spannglied 440 zugeordnete Feder 442 gespannt. Sobald die untere Endlage des Gehäuseteils 10 und des Spanngliedes 440 mit der Kurvenbahn 438 erreicht wurde, wird durch einen am unteren Gehäuseteil 12 vorgesehenen Entkopplungsabschnitt 12d der Kopplungsarm 441 in der in Fig. 6a dargestellten Weise ausgelenkt, so dass der Kontakt zwischen dem Fortsatz 10d und dem Spannglied 440 verloren geht. Dieses wird infolgedessen von der gespannten Feder 442 zusammen mit der Kurvenbahn 438 zurück in die Ausgangslage bewegt. Diese Bewegung erfolgt unabhängig von der Art und Weise, in der der Benutzer das obere Gehäuseteil 10 hinabgedrückt hat. Es kann daher eine nur von baulichen Parametern der Austragvorrichtung abhängige oszillierende Bewegung des Pendelgliedes 436 bewirkt werden, wodurch eine für die konkret verwendete Umwandlungseinrichtung ideale Oszillationsgeschwindigkeit erzielt werden kann. Dies ist beispielsweise von Wert, wenn ein Piezo-biegebalken 444 in der in Fig. 6 gestrichelt dargestellten Weise mit dem Pendelglied 436 zusammenwirkt, da die Nutzung der Eigenfrequenz dieses Biegebalkens zur Minimierung mechanischer Umwandlungsverluste beitragen kann.

Fig. 7 zeigt die bereits beschriebene Umwandlungseinrichtung 50, die auf einer magnetischen Umpolung eines Induktionsorgans 52 basiert.

Alternativ hierzu ist beispielsweise die in Fig. 8 dargestellte Gestaltung einer Umwandlungseinrichtung 250 denkbar, bei der das Pendelglied 36 Schlagbewegungen gegen ein Piezokristall 152 in Reaktion auf eine Betätigung der Austragvorrichtung durchführt. Diese Schlagbewegungen führen zur piezoelektrischen Erzeugung elektrischer Energie.

Die Ausgestaltung der Fig. 9 zeigt als weitere Alternative hierzu in geschnittener und schematischer Ansicht einen Lineargenerator 250. In diesem Falle ist das Pendelorgan 36 mit Permanentmagneten 136a ausgebildet und wird innerhalb einer zum unteren Gehäuseteil 12 ortsfest verbleibenden Spule 252 bewegt, wodurch es zur Erzeugung eines Stroms kommt.

## Patentansprüche

1. Austragvorrichtung für flüssige, pastöse oder pulverförmige Medien mit
- einem Gehäuse (20),
- einer Austragöffnung (10b) zum Austrag des Mediums,
- einem Reservoir (14a) zur Speicherung der Mediums vor dessen Austrag und
- einer gegenüber dem Gehäuse linearbeweglichen Handhabe (10), welche durch eine manuelle bewirkte Hubbewegung die Förderung von Medium vom Reservoir (14a) zur Austragöffnung (10b) bewirkt,
wobei
- die Austragvorrichtung einen elektrischen Verbraucher (20, 24) aufweist und
- die Austragvorrichtung eine Umwandlungseinrichtung (50; 150; 250) aufweist, die zur Umwandlung der an der Handhabe (10) eingebrachten mechanischen Energie in elektrische Energie zur Versorgung des elektrischen Verbrauchers (20, 24) ausgebildet ist,
**dadurch gekennzeichnet, dass**
- zur Zuführung der an der Handhabe (10) eingebrachten mechanischen Energie zur Umwandlungseinrichtung (50; 150; 250) ein Getriebe (30; 130; 230; 330; 430) vorgesehen ist, mittels dessen die lineare Bewegung der Handhabe (10) oder eines Spanngliedes (440) eines mittels der Handhabe (10) spannbaren Federenergiespeichers (440, 442) in eine nicht-rotative oszillierende Bewegung eines Pendelgliedes (36; 136; 236; 336, 338; 436) umgewandelt wird und
- die Umwandlungseinrichtung (50; 150; 250) zur Umwandlung der mechanischen Energie des oszillierenden Pendelgliedes (36; 136; 236; 336; 436) in elektrische Energie ausgebildet ist.

2. Austragvorrichtung nach Anspruch 1,
**dadurch gekennzeichnet, dass**
der Federenergiespeicher (440, 442) mit dem gegenüber dem Gehäuse beweglichen Spannglied (440) und einer zwischen dem Gehäuse (20) und dem Spannglied (440) wirkenden Feder (442) ausgebildet ist, wobei
- das Spannglied (440) während der Hubbewegung mit der Handhabe (10) wirkgekoppelt ist, so dass ein Spannen der Feder (442) bewirkt wird,
- das Spannglied (440) im gespannten Zustand der Feder (442) von der Handhabe (10) automatisch entkoppelbar ist, so dass ein Entspannen der Feder (442) bewirkt wird, und
- das Spannglied (440) während des Entspannens des Feder über das Getriebe (430) mit dem Pendelglied (436) zumindest phasenweise wirkgekoppelt ist, so dass die Bewegung des Spanngliedes (440) die oszillierende Bewegung des Pendelgliedes (436) bewirkt.

3. Austragvorrichtung nach Anspruch 1,
**dadurch gekennzeichnet, dass**
das Pendelglied (338) drehbar oder auslenkbar gelagert ist und mit einer Verzahnung oder Profilierung an der Handhabe, am Spannglied oder einem mit der Handhabe oder mit dem Spannglied in Wirkverbindung stehenden Zwischenglied (334) derart zusammenwirkt, dass die Bewegung der Verzahnung oder Profilierung je Zahn der Verzahnung oder Profilabschnitt der Profilierung dazu führt, dass das Pendelglied (338) in Eingriff mit der Verzahnung oder Profilierung gelangt, durch diese ausgelenkt oder gedreht wird und anschließend wieder außer Eingriff mit der Verzahnung oder Profilierung kommt.

4. Austragvorrichtung nach Anspruch 1,
**dadurch gekennzeichnet, dass**
an der Handhabe (10), am Spannglied (440) oder an einem mit der Handhabe oder mit dem Spannglied in Wirkkopplung stehenden Zwischenglied eine Kurvenbahn (32; 132; 438) vorgesehen ist, an der ein am Pendelglied (36; 136; 436) vorgesehener Fortsatz (38) anliegt, so dass durch eine Bewegung der Handhabe (10) oder des Spanngliedes (440) die Kurvenbahn (32; 132; 438) bewegt wird und der Fortsatz (38) in der Kurvenbahn verlagert wird und dadurch eine Pendelbewegung des Pendelgliedes (36; 136; 436) bewirkt.

5. Austragvorrichtung nach einem der vorstehenden Ansprüche,
**dadurch gekennzeichnet, dass**
die Umwandlungseinrichtung (250) als elektromagnetischer Generator (250) ausgebildet ist, der eine Komponente (36) mit einem Magneten (236a) und eine Komponente (252) mit einem mit dem elektrischen Verbraucher verbundenen Leiter aufweist, wobei eine der Komponenten (252) gehäusefest vorgesehen ist und wobei die andere Komponente am Pendelglied (36) vorgesehen ist.

6. Austragvorrichtung nach einem der vorstehenden Ansprüche,
**dadurch gekennzeichnet, dass**
die Umwandlungseinrichtung (50) aufweist:
- ein Induktionsorgan (52) aus einem magnetisierbaren Material, welches zwischen einem ersten Endbereich (52a) und einem zweiten Endbereich (52b) von einer Spule (60) umwickelt ist, und
- einer Magneteinheit (54) mit einem Magneten (56) und einer ersten und einer zweiten auf Seiten der Magnet-Nordpols (56a) vorgesehenen Nordpolfläche (58a, 58b) und einer ersten und einer zweiten auf Seiten des Magnet-Südpols (56b) vorgesehenen Südpolfläche (59a, 59b),
wobei
- das Induktionsorgan (52) und die Magneteinheit (54) gegeneinander zwischen einer ersten Relativposition und einer zweiten Relativposition beweglich sind, wobei in der ersten Relativposition der erste Endbereich (52a) des Induktionsorgans (52) an der ersten Nordpolfläche (58a) und der zweite Endbereich (52b) des Induktionsorgans (52) an der zweiten Südpolfläche (59a) anliegt und in der zweiten Relativposition der erste Endbereich (52a) des Induktionsorgans (52) an der ersten Südpolfläche (59b) und der zweite Endbereich (52b) des Induktionsorgans (52) an der zweiten Nordpolfläche (58b) anliegt und
- durch Betätigung der Handhabe (10) das Induktionsorgan (52) und die Magneteinheit während eines Betätigungshubes einfach oder mehrfach zwischen den Relativpositionen verlagert werden können.

7. Austragvorrichtung nach einem der vorstehenden Ansprüche,
**dadurch gekennzeichnet, dass**
die Umwandlungseinrichtung einen einseitig gehäusefest eingespannten Piezobiegewandler (338, 444) aufweist, dessen freies Ende durch Betätigung der Handhabe (10) während eines Betätigungshubes einfach oder mehrfach ausgelenkt wird.

8. Austragvorrichtung nach Anspruch 7,
**dadurch gekennzeichnet, dass**
der Piezobiegewandlers (444) als in Reaktion auf eine manuelle Betätigung der Handhabe (10) oszillierend bewegtes Pendelglied (444) ausgebildet ist, wobei die oszillierende Bewegung durch den Federenergiespeicher (440, 442) mit mechanischer Energie versorgt wird und wobei die Feder (442) des Federenergiespeichers (440, 442) derart auf den Piezobiegewandler (444) angepasst ist, dass dieser zumindest phasenweise mit seiner Resonanzfrequenz oszilliert.

9. Austragvorrichtung nach einem der vorstehenden Ansprüche,
**dadurch gekennzeichnet, dass**
die Handhabe entlang einer Betätigungsrichtung gegenüber dem Gehäuse beweglich ist, die mit einer durch eine Austragrichtung des Mediums definierte Haupterstreckungsrichtung der Austragvorrichtung einen Winkel zwischen 70° und 110° einschließt.

## Claims

1. Discharging apparatus for liquid, pasty or pulverulent media, having
- a housing (20),
- a discharging opening (10b) for discharging the medium,
- a reservoir (14a) for storing the medium prior to being discharged, and
- a handle (10), which can be moved linearly in relation to the housing and, by way of a manually induced displacement movement, causes medium to be delivered from the reservoir (14a) to the discharging opening (10b),
wherein
- the discharging apparatus has an electric load (20, 24), and
- the discharging apparatus has a converter (50; 150; 250), by means of which the mechanical energy introduced at the handle (10) is converted into electrical energy for supplying the electric load (20, 24),
**characterized in that**
- the mechanical energy introduced at the handle (10) is fed to the converter (50; 150; 250) by a transmission unit (30; 130; 230; 330; 430), by means of which the linear movement of the handle (10) or of a stressing member (440) of a spring energy store (440, 442), which can be subjected to stressing by means of the handle (10), is converted into a non-rotary oscillating movement of a pendulum member (36; 136; 236; 336, 338; 436), and
- the converter (50; 150; 250) is designed for converting the mechanical energy of the oscillating pendulum member (36; 136; 236; 336; 436) into electrical energy.

2. Discharging apparatus according to Claim 1,
**characterized in that**
the spring energy store (440, 442) is designed with the stressing member (440), which can be moved in relation to the housing, and a spring (442), which acts between the housing (20) and the stressing member (440), wherein
- the stressing member (440) is operatively coupled to the handle (10) during the displacement movement, and this therefore subjects the spring (442) to stressing,
- in the stressed state of the spring (442), the stressing member (440) is uncoupled automatically from the handle (10), and this therefore relieves the spring (442) of stressing, and
- as the spring is being relieved of stressing, the stressing member (440) is operatively coupled to the pendulum member (436), at least in certain phases, via the transmission unit (430), and therefore the movement of the stressing member (440) causes the pendulum member (436) to oscillate.

3. Discharging apparatus according to Claim 1,
**characterized in that**
the pendulum member (338) is mounted such that it can be rotated or deflected, and it interacts with a toothing formation or profiling on the handle, on the stressing member or on an intermediate member (334), which is operatively connected to the handle or to the stressing member, such that the movement of the toothing formation or profiling, for each tooth of the toothing formation or profile portion of the profiling, results in the pendulum member (338) coming into engagement with the toothing formation or profiling, being deflected or rotated thereby and then coming out of engagement with the toothing formation or profiling again.

4. Discharging apparatus according to Claim 1,
**characterized in that**
the handle (10), the stressing member (440) or an intermediate member, which is operatively coupled to the handle or to the stressing member, has provided on it a curved path (32; 132; 438), against which butts an extension (38) provided on the pendulum member (36; 136; 436), and therefore a movement of the handle (10) or of the stressing member (440) moves the curved path (32; 132; 438) and the extension (38) is shifted in the curved path, and this causes a pendulum movement of the pendulum member (36; 136; 436).

5. Discharging apparatus according to one of the preceding claims,
**characterized in that**
the converter (250) is designed as an electromagnetic generator (250), which has a component (36) with a magnet (236a) and a component (252) with a conductor connected to the electric load, wherein one of the components (252) is mounted on the housing, and wherein the other component is provided on the pendulum member (36).

6. Discharging apparatus according to one of the preceding claims,
**characterized in that**
the converter (50) has:
- an induction mechanism (52) made of a magnetizable material, which has a coil (60) wound around it between a first end region (52a) and a second end region (52b), and
- a magnet unit (54) with a magnet (56) and a first and a second north-pole surface (58a, 58b), provided on the north pole (56a) of the magnet, and a first and a second south-pole surface (59a, 59b), provided on the south pole (56b) of the magnet,
wherein
- the induction mechanism (52) and the magnet unit (54) can be moved in relation to one another between a first relative position and a second relative position, wherein, in the first relative position, the first end region (52a) of the induction mechanism (52) butts against the first north-pole surface (58a) and the second end region (52b) of the induction mechanism (52) butts against the second south-pole surface (59a) and, in the second relative position, the first end region (52a) of the induction mechanism (52) butts against the first south-pole surface (59b) and the second end region (52b) of the induction mechanism (52) butts against the second north-pole surface (58b), and
- by actuation of the handle (10), the induction mechanism (52) and the magnet unit can be shifted between the relative positions one or more times during an actuating displacement.

7. Discharging apparatus according to one of the preceding claims,
**characterized in that**
the converter has a piezoelectric bending transducer (338, 444), of which one end is clamped in the housing and the free end is deflected one or more times by actuation of the handle (10) during an actuating displacement.

8. Discharging apparatus according to Claim 7,
**characterized in that**
the piezoelectric bending transducer (444) is designed as a pendulum member (444) which oscillates in reaction to manual actuation of the handle (10), wherein the oscillating movement is supplied with mechanical energy by the spring energy store (440, 442), and wherein the spring (442) of the spring energy store (440, 442) is adapted to the piezoelectric bending transducer (444) such that the latter oscillates at its resonance frequency at least in certain phases.

9. Discharging apparatus according to one of the preceding claims,
**characterized in that**
the handle can be moved in relation to the housing in an actuating direction which encloses an angle between 70° and 110° with a main direction of extent of the discharging apparatus, this latter direction being defined by a discharging direction of the medium.

## Revendications

1. Dispositif de distribution pour milieux liquides, pâteux ou pulvérulents, comprenant
- un boîtier (20),
- une ouverture de distribution (10b) pour distribuer le milieu,
- un réservoir (14a) pour stocker le milieu avant sa distribution et
- une manette (10) déplaçable linéairement par rapport au boîtier, qui, par un mouvement de course effectué manuellement, provoque le déplacement de milieu depuis le réservoir (14a) vers l'ouverture de distribution (10b),
dans lequel
- le dispositif de distribution présente un consommateur électrique (20, 24), et
- le dispositif de distribution présente un dispositif de conversion (50 ; 150 ; 250) qui est réalisé pour convertir l'énergie mécanique introduite au niveau de la manette (10) en énergie électrique pour l'alimentation du consommateur électrique (20, 24),
**caractérisé en ce que**
- pour le transfert de l'énergie mécanique introduite au niveau de la manette (10) au dispositif de conversion (50 ; 150 ; 250), il est prévu un mécanisme (30 ; 130 ; 230 ; 330, 430) au moyen duquel le mouvement linéaire de la manette (10) ou d'un organe de tensionnement (440) d'un accumulateur d'énergie à ressort (440, 442) pouvant être tendu au moyen de la manette (10) est converti en un mouvement non rotatif d'oscillation d'un organe de pendule (36 ; 136 ; 236 ; 336, 338 ; 436) et
- le dispositif de conversion (50 ; 150 ; 250) est réalisé pour convertir l'énergie mécanique de l'organe de pendule oscillant (36 ; 136 ; 236 ; 336 ; 436) en énergie électrique.

2. Dispositif de distribution selon la revendication 1,
**caractérisé en ce que**
l'accumulateur d'énergie à ressort (440, 442) est réalisé avec l'organe de tensionnement (440) déplaçable par rapport au boîtier et avec un ressort (442) agissant entre le boîtier (20) et l'organe de tensionnement (440),
- l'organe de tensionnement (440) étant accouplé fonctionnellement avec la manette (10) pendant le mouvement de levage de telle sorte que le ressort (442) soit tendu,
- l'organe de tensionnement (440), dans l'état tendu du ressort (442), pouvant être désaccouplé automatiquement de la manette (10), de manière à relâcher le ressort (442), et
- l'organe de tensionnement (440), pendant la détente du ressort, étant accouplé fonctionnellement par le biais du mécanisme (430) à l'organe de pendule (436) au moins par moments de telle sorte que le mouvement de l'organe de tensionnement (440) provoque le mouvement d'oscillation de l'organe de pendule (436) .

3. Dispositif de distribution selon la revendication 1,
**caractérisé en ce que**
l'organe de pendule (338) est supporté de manière rotative ou de manière à pouvoir être dévié et coopère avec une denture ou un profilage sur la manette, sur l'organe de tensionnement ou sur un organe intermédiaire (334) en liaison fonctionnelle avec la manette ou avec l'organe de tensionnement, de telle sorte que le mouvement de la denture ou du profilage pour chaque dent de la denture ou de la portion profilée du profilage fasse en sorte que l'organe de pendule (338) s'engage avec la denture ou le profilage, soit dévié ou tourné par celle-ci ou celui-ci et finalement se désengage à nouveau d'avec la denture ou le profilage.

4. Dispositif de distribution selon la revendication 1,
**caractérisé en ce que**
sur la manette (10), sur l'organe de tensionnement (440) ou sur un organe intermédiaire en liaison fonctionnelle avec la manette ou avec l'organe de tensionnement, est prévue une voie courbe (32 ; 132 ; 438) contre laquelle s'applique une saillie (38) prévue sur l'organe de pendule (36 ; 136 ; 436), de telle sorte que par un déplacement de la manette (10) ou de l'organe de tensionnement (440), la voie courbe (32 ; 132 ; 438) soit déplacée et la saillie (38) soit décalée dans la voie courbe et provoque de ce fait un mouvement pendulaire de l'organe de pendule (36 ; 136 ; 436) .

5. Dispositif de distribution selon l'une quelconque des revendications précédentes,
**caractérisé en ce que**
le dispositif de conversion (250) est réalisé sous forme de générateur électromagnétique (250) qui présente un composant (36) avec un aimant (236a) et un composant (252) avec un conducteur connecté au consommateur électrique, l'un des composants (252) étant prévu de manière fixée au boîtier et l'autre composant étant prévu sur l'organe de pendule (36).

6. Dispositif de distribution selon l'une quelconque des revendications précédentes,
**caractérisé en ce que**
le dispositif de conversion (50) présente :
- un organe d'induction (52) constitué d'un matériau magnétisable, qui est enveloppé entre une première région d'extrémité (52a) et une deuxième région d'extrémité (52b) par une bobine (60) et
- une unité d'aimant (54) avec un aimant (56) et une première et une deuxième surface de pôle nord (58a, 58b) prévues du côté du pôle nord magnétique (56a) et une première et une deuxième surface de pôle sud (59a, 59b) prévues du côté du pôle sud magnétique (56b),
- l'organe d'induction (52) et l'unité magnétique (54) pouvant être déplacés l'un par rapport à l'autre entre une première position relative et une deuxième position relative, dans la première position relative, la première région d'extrémité (52a) de l'organe d'induction (52) s'appliquant contre la première surface de pôle nord (58a) et la deuxième région d'extrémité (52b) de l'organe d'induction (52) s'appliquant contre la deuxième surface de pôle sud (59a) et dans la deuxième position relative, la première région d'extrémité (52a) de l'organe d'induction (52) s'appliquant contre la première surface de pôle sud (59b) et la deuxième région d'extrémité (52b) de l'organe d'induction (52) s'appliquant contre la deuxième surface de pôle nord (58b), et
- par actionnement de la manette (10), l'organe d'induction (52) et l'unité magnétique pouvant être déplacés pendant une course d'actionnement une fois ou plusieurs fois entre les positions relatives.

7. Dispositif de distribution selon l'une quelconque des revendications précédentes,
**caractérisé en ce que**
le dispositif de conversion présente un transducteur piézoélectrique à flexion (338, 444) serré fixement au boîtier d'un côté, dont l'extrémité libre est déviée une ou plusieurs fois par actionnement de la manette (10) pendant une course d'actionnement.

8. Dispositif de distribution selon la revendication 7, **caractérisé en ce que** le transducteur piézoélectrique à flexion (444) est réalisé sous forme d'organe de pendule (444) déplacé en oscillation en réaction à un actionnement manuel de la manette (10), le mouvement d'oscillation étant alimenté en énergie mécanique par l'accumulateur d'énergie à ressort (440, 442) et le ressort (442) de l'accumulateur d'énergie à ressort (440, 442) étant adapté au transducteur piézoélectrique à flexion (444) de telle sorte que celui-ci oscille au moins par moments à sa fréquence de résonance.

9. Dispositif de distribution selon l'une quelconque des revendications précédentes, **caractérisé en ce que**
la manette peut être déplacée le long d'une direction d'actionnement par rapport au boîtier, laquelle forme un angle compris entre 70° et 110° avec une direction d'étendue principale définie par une direction de distribution de milieu du dispositif de distribution.
